# EUROPEAN PATENT APPLICATION

(11) **EP 4 674 354 A1**
(43) Date of publication of application: **07.01.2026**
(21) Application number: 25187028.3
(22) Date of filing: 02.07.2025
(51) Int. Cl.: A61B 8/06, A61B 8/00

(54) **ULTRASOUND DIAGNOSTIC APPARATUS, MEDICAL INFORMATION PROCESSING APPARATUS, AND MEDICAL INFORMATION PROCESSING METHOD**

(30) Priority: 02.07.2024 JP 2024106695; 30.06.2025 JP 2025110953
(71) Applicant: Canon Medical Systems Corporation, Tochigi 324-0036 (JP)
(72) Inventor: IKEDA, Hayato, Otawara-shi, 324-0036 (JP); KAKEE, Akihiro, Otawara-shi, 324-0036 (JP); IWASAKI, Ryosuke, Otawara-shi, 324-0036 (JP)
(74) Representative: Marks & Clerk LLP

(57) **Abstract**

An ultrasound diagnostic apparatus (110) according to an embodiment includes a processing circuit (150). The processing circuit (150) sequentially acquires first ultrasound data (1a, 1b, 1c) representing a fluid in a body of a subject in a time direction based on an ultrasound reception signal received by an ultrasound probe (105), executes high-definition processing that locally extracts or emphasizes the fluid represented by the first ultrasound data (1a, 1b, 1c) to acquire second ultrasound data, composites a first ultrasound image (4) that is based on the first ultrasound data (1a, 1b, 1c) and a second ultrasound image (3) that is based on the second ultrasound data, and displays a composite image (5) obtained by the composition.

## Description

### FIELD

Embodiments described herein relate generally to an ultrasound diagnostic apparatus, a medical information processing apparatus, and a medical information processing method.

### BACKGROUND

Conventionally, techniques to improve the image quality of ultrasound images containing minute flow paths and fluids (for example, blood flow, contrast media, and the like) are known. For example, Japanese Patent Application Laid-open No. 2001-178720 discloses that an image representing the boundaries of flow paths (for example, the shape of blood vessels) is generated by removing clutter contained in a consecutive ultrasound receiver signal in a certain time and averaging the signal of a fluid after removal of the clutter in a certain time. In addition, Translation of PCT Application No. 2019-526350 discloses that super-resolution processing is performed that improves the resolution (pixel density) of an ultrasound image and sharpens the peak of a speckle pattern in the ultrasound image.

### SUMMARY OF INVENTION

A medical information processing apparatus provided according to an aspect of the present invention includes a processing circuit. The processing circuit is configured to acquire a plurality of pieces of first ultrasound data of a subject; to execute high-definition processing on the pieces of first ultrasound data; and to composite second ultrasound data based on a result executed by the execution unit and data to be composited that is at least one of the first ultrasound data and data obtained by performing certain processing on the first ultrasound data with each other.

The processing circuit may detect a characteristic amount of clutter; and determine a blend ratio between the second ultrasound data and the data to be composited based on the characteristic amount. The processing circuit may composite the second ultrasound data and the data to be composited with each other based on the blend ratio.

The processing circuit may detect the characteristic amount based on a filter coefficient in removal processing for the clutter.

The processing circuit may increase the blend ratio of the second ultrasound data when a difference between the filter coefficient corresponding to a first eigenvalue and the filter coefficient corresponding to a second eigenvalue is large compared to a case when the difference is small.

The processing circuit may detect the characteristic amount based on a shape of distribution of the filter coefficient.

The processing circuit may detect the characteristic amount based on a temporal change in the filter coefficient.

The processing circuit may detect signal intensity of the data to be composited or the second ultrasound data as the characteristic amount.

The processing circuit may detect a change in the data to be composited or the second ultrasound data in a frame direction as the characteristic amount.

The processing circuit may detect a change in phase information of the data to be composited or the second ultrasound data in a frame direction as the characteristic amount.

The processing circuit may detect the characteristic amount for each of a plurality of divided spatial regions.

The processing circuit may detect the characteristic amount in accordance with a region where clutter is detected.

The processing circuit may detect the characteristic amount for each of a plurality of spatial regions divided in a time direction.

The processing circuit may include a display controller that displays composite data composited by the composition unit on a display unit.

An ultrasound diagnostic apparatus provided according to an aspect of the present invention includes a transmitter and receiver and a processing circuit. The transmitter and receiver is configured to cause an ultrasound probe to execute an ultrasound scan of a subject; The processing circuit is configured to acquire a plurality of pieces of first ultrasound data of the subject obtained by the ultrasound scan;
to execute high-definition processing on the pieces of first ultrasound data; and to composite second ultrasound data based on a result executed by the execution unit and data to be composited that is at least one of the first ultrasound data and data obtained by performing certain processing on the first ultrasound data with each other.

A medical information processing method provided according to an aspect of the present invention includes:
acquiring a plurality of pieces of first ultrasound data of a subject;
executing high-definition processing on the pieces of first ultrasound data; and
composing second ultrasound data based on a result executed by an execution unit and data to be composited that is at least one of the first ultrasound data and data obtained by performing certain processing on the first ultrasound data with each other.

An ultrasound diagnostic apparatus provided according to an aspect of the present invention includes a processing circuit. The processing circuit is configured to sequentially acquire first ultrasound data representing a fluid in a body of a subject in a time direction based on an ultrasound reception signal received by an ultrasound probe; to execute high-definition processing that locally extracts or emphasizes the fluid represented by the first ultrasound data to acquire second ultrasound data; to composite a first ultrasound image based on the first ultrasound data and a second ultrasound image based on the second ultrasound data with each other; and to display a composite image obtained by the composition unit.

The processing circuit may detect a characteristic amount of clutter; and
determine a composition ratio at which the first ultrasound data and the second ultrasound data are composited with each other based on the characteristic amount of clutter. The processing circuit may composite the first ultrasound image and the second ultrasound image with each other based on the composition ratio determined by the determination unit.

The processing circuit may detect a filter coefficient of an adaptive filter as the characteristic amount of clutter, the adaptive filter reducing clutter contained in an input signal, and
the processing circuit may determine the composition ratio based on the filter coefficient detected by the detector.

The adaptive filter may decompose the input signal into a plurality of eigenvalues, and
the processing circuit may detect distribution of the eigenvalues decomposed by the adaptive filter as the characteristic amount of clutter.

The processing circuit may detect a difference between two eigenvalues among the eigenvalues as the characteristic amount of clutter, and determine the composition ratio such that a ratio of the first ultrasound image to the second ultrasound image becomes larger as the difference between the eigenvalues becomes smaller.

The processing circuit may detect signal intensity of at least one ultrasound data of the first ultrasound data and the second ultrasound data as the characteristic amount of clutter.

The processing circuit may detect a temporal change in signal intensity of at least one ultrasound data of the first ultrasound data and the second ultrasound data as the characteristic amount of clutter.

The processing circuit may detect a change in phase information of at least one ultrasound data of the first ultrasound data and the second ultrasound data as the characteristic amount of clutter.

The processing circuit may detect the characteristic amount of clutter for each of positions of frames in at least one ultrasound data of the first ultrasound data and the second ultrasound data, determine the composition ratio of each position based on the characteristic amount of clutter detected for each of the positions, and perform composition of the first ultrasound image and the second ultrasound image at each position based on the composition ratio of each position determined by the determination unit.

The processing circuit may receive an operation to adjust a composition ratio at which the first ultrasound image and the second ultrasound image are composited with each other, and may composite the first ultrasound image and the second ultrasound image with each other based on the composition ratio.

The processing circuit may detect a characteristic amount of clutter, may control whether the first ultrasound image and the second ultrasound image are composited with each other based on the characteristic amount of clutter, and may display the composite image when the first ultrasound image and the second ultrasound image are composited with each other and display the second ultrasound image when the first ultrasound image and the second ultrasound image are not composited with each other.

The processing circuit may detect a characteristic amount of clutter, may control whether the first ultrasound image and the second ultrasound image are composited with each other based on the characteristic amount of clutter, and may display the composite image when the first ultrasound image and the second ultrasound image are composited with each other and display the first ultrasound image when the first ultrasound image and the second ultrasound image are not composited with each other.

A medical information processing apparatus provided according to an aspect of the present invention includes a processing circuit. The processing circuit is configured to sequentially acquire first ultrasound data representing a fluid in a body of a subject in a time direction based on an ultrasound reception signal received by an ultrasound probe; to execute high-definition processing that locally extracts or emphasizes the fluid represented by the first ultrasound data to acquire second ultrasound data; to composite a first ultrasound image that is based on the first ultrasound data and a second ultrasound image that is based on the second ultrasound data; and to display the composite image.

A medical information processing method provided according to an aspect of the present invention causes a computer to sequentially acquire first ultrasound data representing a fluid in a body of a subject in a time direction based on an ultrasound reception signal received by an ultrasound probe; to execute high-definition processing that locally extracts or emphasizes the fluid represented by the first ultrasound data to acquire second ultrasound data; to composite a first ultrasound image that is based on the first ultrasound data and a second ultrasound image that is based on the second ultrasound data; and to display the composite image.

### BRIEF DESCRIPTION OF THE DRAWINGS

FIG. 1 is a diagram of an example of the configuration of an ultrasound diagnostic apparatus according to an embodiment;
FIG. 2 is a flowchart of the flow of processing performed by the ultrasound diagnostic apparatus illustrated in FIG. 1;
FIG. 3 is a diagram illustrating how an acquisition function acquires first ultrasound data representing a fluid in the body of a subject based on an ultrasound reception signal received by an ultrasound probe in an ultrasound diagnostic apparatus according to a first embodiment;
FIG. 4 is a diagram illustrating how an execution function generates addition data (data representing a single image) by adding/time-averaging (persistence) a plurality of pieces of frame data or the like consecutive in a time direction and for which a clutter component has been reduced by processing at Step S110 every certain number of frames in the ultrasound diagnostic apparatus according to the first embodiment;
FIG. 5 is a diagram illustrating how the execution function places a kernel at a position of interest in a frame (image) represented by the addition data, and compares the magnitude relation between a signal value corresponding to the position of interest and a signal value corresponding to each position in the range where the kernel is placed (around the position of interest) in the ultrasound diagnostic apparatus according to the first embodiment;
FIG. 6 is a diagram illustrating how a first ultrasound image based on a blood flow signal and a second ultrasound image based on a high-definition signal are composited with each other at a certain composition ratio to generate a composite image in the ultrasound diagnostic apparatus according to the first embodiment;
FIG. 7 is a diagram of a blood flow image (a first ultrasound image illustrated in FIG. 3 and FIG. 4) in the ultrasound diagnostic apparatus according to the first embodiment;
FIG. 8 is a diagram of a high-definition image (a second ultrasound image illustrated in FIG. 3 and FIG. 4) in the ultrasound diagnostic apparatus according to the first embodiment;
FIG. 9 is a diagram of a composite image (a composite image illustrated in FIG. 3 and FIG. 4) in the ultrasound diagnostic apparatus according to the first embodiment;
FIG. 10 is a diagram illustrating how a display control function displays a graphical user interface (GUI) including two types of sliders in addition to the composite image on a display in the ultrasound diagnostic apparatus according to the first embodiment;
FIG. 11 is a diagram illustrating how a blood flow image obtained when the clutter component remains in the first ultrasound data is affected by clutter in a background description for an ultrasound diagnostic apparatus according to a second embodiment;
FIG. 12 is a diagram illustrating how the connection of blood flow cannot be expressed in the high-definition image (the second ultrasound image) when high-definition processing is executed on the first ultrasound data as illustrated in FIG. 11 in the background description for the ultrasound diagnostic apparatus according to the second embodiment;
FIG. 13 is a diagram of an example of the configuration of the ultrasound diagnostic apparatus according to the second embodiment;
FIG. 14 is a flowchart illustrating the flow of processing performed by the ultrasound diagnostic apparatus according to the second embodiment;
FIG. 15 is a diagram illustrating how the first ultrasound image (the blood flow image) and the second ultrasound image (the high-definition image) are composited with each other based on a composition ratio adaptively adjusted and determined by a determination function to generate a composite image in the ultrasound diagnostic apparatus according to the second embodiment;
FIG. 16 to FIG. 18 are diagrams illustrating examples of the distribution of a filter coefficient in the ultrasound diagnostic apparatus according to the second embodiment;
FIG. 19 is a diagram illustrating how an eigenvalue is plotted against an eigenvalue number in the ultrasound diagnostic apparatus according to the second embodiment;
FIG. 20 is a diagram illustrating how a detection function spatially divides a frame of at least one ultrasound data of the first ultrasound data and the second ultrasound data into a plurality of regions, and detects a characteristic amount of clutter for each of the spatially divided regions in the ultrasound diagnostic apparatus according to the second embodiment;
FIG. 21 is a diagram illustrating how the acquisition function acquires the first ultrasound data representing the fluid in the body of the subject based on the ultrasound reception signal received by the ultrasound probe in the ultrasound diagnostic apparatus according to the second embodiment; and
FIG. 22 is a diagram illustrating an example of possible high-definition processing in the ultrasound diagnostic apparatus according to the second embodiment.

### DETAILED DESCRIPTION

An ultrasound diagnostic apparatus according to an embodiment includes a processing circuit. The processing circuit sequentially acquires first ultrasound data representing a fluid in a body of a subject in a time direction based on an ultrasound reception signal received by an ultrasound probe, executes high-definition processing that locally extracts or emphasizes the fluid represented by the first ultrasound data to acquire second ultrasound data, composites a first ultrasound image based on the first ultrasound data and a second ultrasound image based on the second ultrasound data with each other, and displays a composite image obtained by the composition.

Embodiments of an ultrasound diagnostic apparatus, a medical information processing apparatus, and a medical information processing method according to the present application will be described below in detail with reference to the accompanying drawings. Note that the ultrasound diagnostic apparatus, the medical information processing apparatus, and the medical information processing method according to the present application are not limited by the embodiments illustrated below.

### First Embodiment

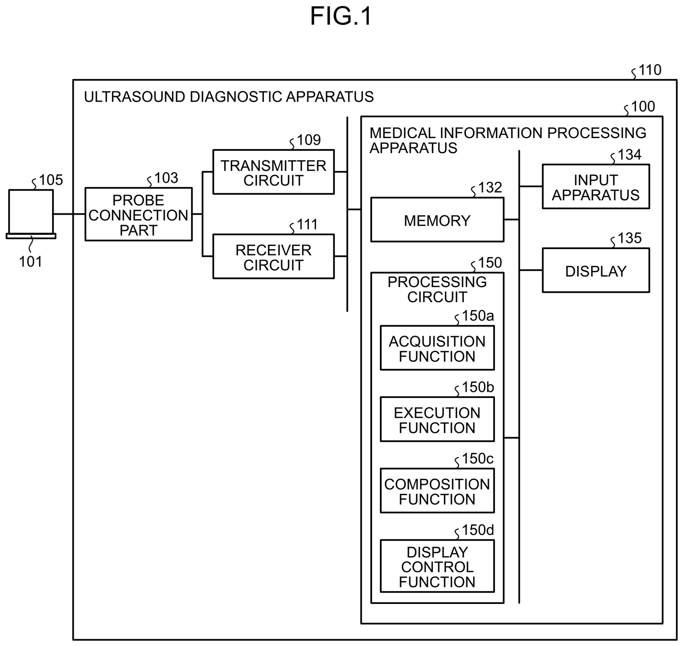

First, an example of the configuration of a medical information processing apparatus according to an embodiment will be described using FIG. 1. This ultrasound diagnostic apparatus 110 is an apparatus that generates ultrasound image data based on a reception signal (a reflected wave signal) received from an ultrasound probe 105. The ultrasound diagnostic apparatus 110 illustrated in FIG. 1 is an apparatus that can generate two-dimensional ultrasound image data based on a reception signal received from a one-dimensional ultrasound probe 105 (an ultrasound probe in which transducer elements are arranged in one dimension) and can generate three-dimensional ultrasound data based on a reception signal received from a two-dimensional ultrasound probe 105 (an ultrasound probe in which transducer elements are arranged in two dimensions).

The ultrasound probe 105 transmits an ultrasound wave to a subject and receives the ultrasound wave reflected by the subject. The ultrasound probe 105 is, for example, an electronic scanning probe, having a plurality of transducer elements 101 arranged in one dimension or two dimensions at its tip. The transducer element 101 is a piezoelectric element (an electromechanical conversion element) that performs mutual conversion between an electric signal (a voltage pulse signal) and an ultrasound wave (an acoustic wave). The ultrasound probe 105 transmits ultrasound waves from the transducer elements 101 to the subject and receives reflected ultrasound waves from the subject by the transducer elements 101. A reflected acoustic wave reflects differences in acoustic impedance within the subject. Note that the reflected ultrasound wave when a transmitted ultrasound pulse is reflected on a moving blood flow or a surface such as the heart wall is subjected to a frequency shift because of the Doppler effect, depending on the velocity signal component of a moving body with respect to an ultrasound transmission direction.

The ultrasound diagnostic apparatus 110 includes a probe connection part 103, a transmitter circuit 109, a receiver circuit 111, and a medical information processing apparatus 100.

The probe connection part 103 connects the ultrasound probe 105 and performs transmission and reception of ultrasound signals (electric signals) to and from the ultrasound probe 105. The means of connection of the ultrasound probe 105 by the probe connection part 103 may be either wired or wireless. In the wired case, the probe connection part 103 has a connector part (receptacle) to which a connector (plug) of the ultrasound probe 105 is connected. In the wireless case, it has a communication unit that performs wireless communication with the ultrasound probe 105.

The transmitter circuit 109 is a transmitter that outputs pulse signals (drive signals) to the transducer elements 101. By applying pulse signals with time differences to the transducer elements 101, ultrasound waves with different delay times are transmitted from the transducer elements 101 to form a transmission ultrasound beam. The direction and focus of the transmission ultrasound beam can be controlled by selectively changing the transducer elements 101 to which the pulse signals are applied (that is, the transducer elements 101 to be driven) or by changing the delay time (application timing) of the pulse signals. By sequentially changing the direction and focus of this transmission ultrasound beam, an observation area inside the subject is scanned. By changing the delay time of the pulse signals, the transmission ultrasound beam may be formed that is a plane wave (its focus is far away) or a diffusion wave (its focus point is opposite to the transducer elements 101 in the ultrasound transmission direction). Alternatively, the transmission ultrasound beam may be formed using one transducer element or part of the transducer elements 101. The transmitter circuit 109 transmits the pulse signal with a certain drive waveform to the transducer element 101 to generate a transmission ultrasound wave having a certain transmission waveform at the transducer element 101.

The receiver circuit 111 is a receiver that receives input of the electric signal output from the transducer element 101, which has received the reflected ultrasound wave, as a reception signal. The reception signal is input to a processing circuit 150. Note that in the present embodiment, both an analog signal output from the transducer element 101 and digital data sampled (digitally converted) from it are referred to as the reception signal without any particular distinction.

The medical information processing apparatus 100 is connected to the transmitter circuit 109 and the receiver circuit 111, and executes processing on the signal received from the receiver circuit 111 and control of the transmitter circuit 109. The medical information processing apparatus 100 includes the processing circuit 150, a memory 132, an input apparatus 134, and a display 135.

The memory 132 includes a semiconductor memory element such as a random access memory (RAM) and a flash memory, a hard disk, an optical disc, or the like. The memory 132 is a memory storing therein data such as image data for display generated by the processing circuit 150. The memory 132 can also store therein the reception signal (the reflected wave signal) output by the receiver circuit 111. In addition, the memory 132 stores therein control programs for performing ultrasound transmission and reception, image processing, and display processing, diagnostic information (for example, patient IDs, opinions by doctors, and the like), and various data such as diagnostic protocols and various body marks as needed.

The input apparatus 134 receives various instructions and information input from the operator. The input apparatus 134 includes, for example, an input interface apparatus such as a trackball, a switch button, a mouse, a keyboard, a touchpad for performing input operation by touching an operating surface, a touch monitor that integrates a display screen and a touchpad, a non-contact input circuit including an optical sensor, or a voice input circuit. Note that the input interface apparatus is connected to the processing circuit 150 described below, and converts an input operation received from the operator into an electric signal and outputs it to the processing circuit 150. Note that in the present specification, the input interface apparatus is not limited only to those including physical operating components such as a mouse and a keyboard. For example, an electric signal processing circuit that receives an electric signal corresponding to an input operation from an external input device provided separately from the apparatus and outputs this electric signal to the processing circuit 150 is also included in examples of the input interface apparatus. The input apparatus 134 is an example of an operating unit.

The display 135 displays a graphical user interface (GUI) for receiving input of imaging conditions and various images under the control of the processing circuit 150. The display 135 includes, for example, a display interface apparatus such as a liquid crystal display.

The processing circuit 150 controls each part of the ultrasound diagnostic apparatus 110, thereby controlling the entire ultrasound diagnostic apparatus 110. For example, the processing circuit 150 executes computer programs stored in the memory 132, thereby functioning as an acquisition function 150a, an execution function 150b, a composition function 150c, and a display control function 150d. The acquisition function 150a, the execution function 150b, the composition function 150c, and the display control function 150d are examples of an acquisition unit, an execution unit, a composition unit, and a display controller, respectively. Note that in FIG. 1, the processing circuit 150 is described as being implemented by a single, but a plurality of independent processors may be combined to implement it by many. It may include independent circuits dedicated to specific functions, such as an application specific integrated circuit (ASIC).

The term "processor" used in the above description means, for example, a circuit such as a central processing unit (CPU), a graphical processing unit (GPU), an application specific integrated circuit (ASIC), or a programmable logic device (for example, a simple programmable logic device (SPLD), a complex programmable logic device (CPLD), or a field programmable gate array (FPGA)). The processor reads and executes the computer program stored in the memory 132 to implement the functions.

The ultrasound diagnostic apparatus 110 configured as described above performs the following processing.

(Processing 1) Acquisition processing that sequentially acquires first ultrasound data representing a fluid in a body of a subject in a time direction based on an ultrasound reception signal received by the ultrasound probe 105

(Processing 2) Execution processing that executes high-definition processing that locally extracts or emphasizes the fluid represented by the first ultrasound data to acquire second ultrasound data

(Processing 3) Composition processing that composites a first ultrasound image based on the first ultrasound data and a second ultrasound image based on the second ultrasound data with each other

Processing 2 corresponds to processing that improves the image quality (high-definition processing) for the first ultrasound data (for example, power Doppler data) representing the fluid (for example, blood flow) in the body of the subject obtained by Processing 1. Specifically, Processing 2 is processing that improves the visibility (contrast and sharpness) of blood flow by analyzing the sequentially acquired first ultrasound data over a certain time and extracting or emphasizing a signal with a relatively large signal value (a local peak signal) in a local range. The position of the local peak signal identified in each frame is discrete. Therefore, there is a problem of trade-off: as an analysis time in Processing 2 becomes longer (as the number of analysis frames becomes larger), the position of the local peak signal can be expressed to be more continuous (the connection of blood flow can be expressed more), but real-time properties become worse accordingly. In other words, if real-time properties are emphasized, the position of the local peak signal will be discontinuous, and the image quality of the ultrasound image based on the second ultrasound data acquired by Processing 2 (the second ultrasound image) will be degraded (the connection of blood flow cannot be expressed).

Processing 3 is processing to compensate for the image quality of the ultrasound image based on the second ultrasound data due to the emphasis on real-time properties by the first ultrasound image based on the first ultrasound data. The first ultrasound image is more sensitive than the second ultrasound image. In Processing 3, the first ultrasound image and the second ultrasound image are composited with each other. This reduces the problem of trade-off described above caused by the processing that improves the image quality of the ultrasound image.

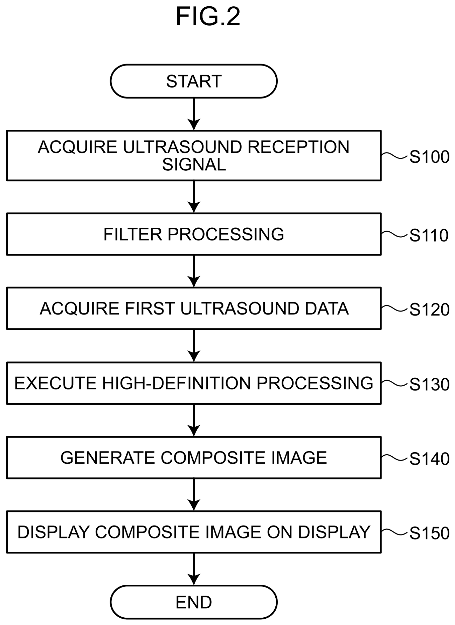

The details of the operation of the ultrasound diagnostic apparatus 110 will be described below with reference to FIG. 2 to FIG. 9. FIG. 2 is a flowchart illustrating the flow of processing performed by the medical information processing apparatus 100 according to the first embodiment.

The acquisition function 150a controls the transmitter circuit 109 and the receiver circuit 111 to cause the ultrasound probe 105 to execute an ultrasound scan, and acquires a reception signal with a plurality of frames (for example, CH data) (Step S100). Specifically, the acquisition function 150a starts control to collect a plurality of pieces of frame data consecutive in the time direction obtained by the execution of the ultrasound scan (a plurality of pieces of frame data within a certain time) at a certain frame rate. The frame data contains an amplitude value (a signal value) of a signal corresponding to each pixel constituting one image (frame).

The acquisition function 150a performs phasing addition and/or quadrature detection processing on the collected reception signals. The phasing addition processing is processing to add together the reception signals of the transducer elements 101 with different delay times and weights for each transducer element 101, and is also called delay and sum (DAS) beamforming. The quadrature detection processing is processing to convert the reception signals into in-phase signals and quadrature signals in the baseband to acquire measurement data representing IQ data or the absolute value of IQ data or the like. Note that apart from the above, processing using adaptive beamforming, model-based processing, and machine learning and the like may be performed on the reception signals.

When the pieces of frame data are collected, the acquisition function 150a estimates the displacement amount of tissue due to body motion of the subject and the like between the pieces of frame data, and corrects each frame data based on an estimated result. Specifically, the acquisition function 150a calculates the displacement amount of tissue due to body motion and the like between the frames from the pieces of frame data, and corrects the frame data based on the calculated displacement amount.

The acquisition function 150a performs filter processing that extracts blood flow-derived information contained in the input signal (reduces clutter) (Step S110). For example, the acquisition function 150a applies a moving target indicator (MTI) filter to each collected frame data (the input signal). This reduces information derived from tissue that is stationary between frames or tissue with little movement (clutter), and extracts information derived from blood flow (a blood flow signal component). As the MTI filter, a filter with fixed filter information, such as a Butterworth type infinite impulse response (IIR) filter or a polynomial regression filter, may be used. The MTI filter may be an adaptive filter that changes its filter coefficient in accordance with the input signal using eigenvalue decomposition or singular value decomposition.

The acquisition function 150a can also reduce the tissue-derived information and extract the blood flow-derived information by decomposing the frame data into a plurality of bases by eigenvalue decomposition, singular value decomposition, or the like and extracting a specific base. The acquisition function 150a can also determine a velocity vector for each coordinate in the reception signal data and determine a blood flow vector representing the magnitude and direction of blood flow by using a method such as the vector Doppler method, the speckle tracking method, or the vector flow mapping method. Apart from the methods exemplified here, methods that can extract the blood flow-derived information or reduce the tissue-derived information contained in the frame data (measurement data) can be employed. For example, for the filter processing, a wall filter may be used to reduce tissue components from the ultrasound frame data. The wall filter is used to effectively reduce low-frequency tissue components (clutter) mainly caused by body motion and the like from the ultrasound reception signal (the frame data) and to extract blood flow components (Doppler components). Note that the acquisition function 150a can also perform envelope detection processing, logarithmic compression processing, or the like on the measurement data to generate B-mode data in which signal intensity at each point in the observation area is expressed in terms of brightness.

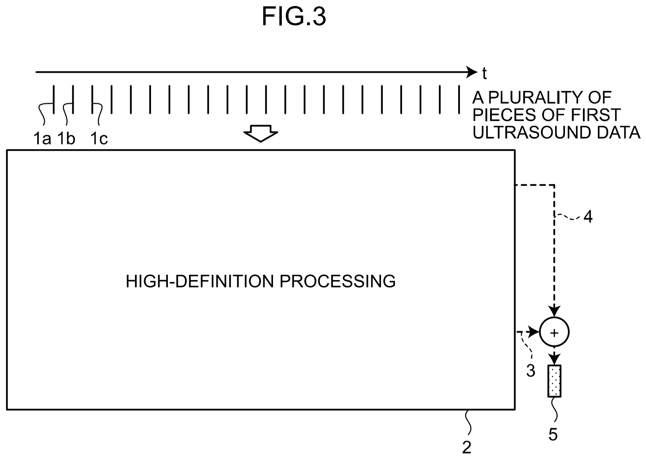

For example, as illustrated in FIG. 3, the acquisition function 150a acquires the first ultrasound data representing the fluid in the body of the subject based on the ultrasound reception signal received by the ultrasound probe 105. Specifically, when the filter processing at Step S110 is performed, the acquisition function 150a sequentially acquires a plurality of pieces of frame data 1a, 1b, 1c, ... consecutive in the time direction as the first ultrasound data (Step S120). Each frame data is power Doppler data and contains an amplitude value (a power signal value) of a blood flow signal (a fluid signal) corresponding to each pixel constituting one image (frame).

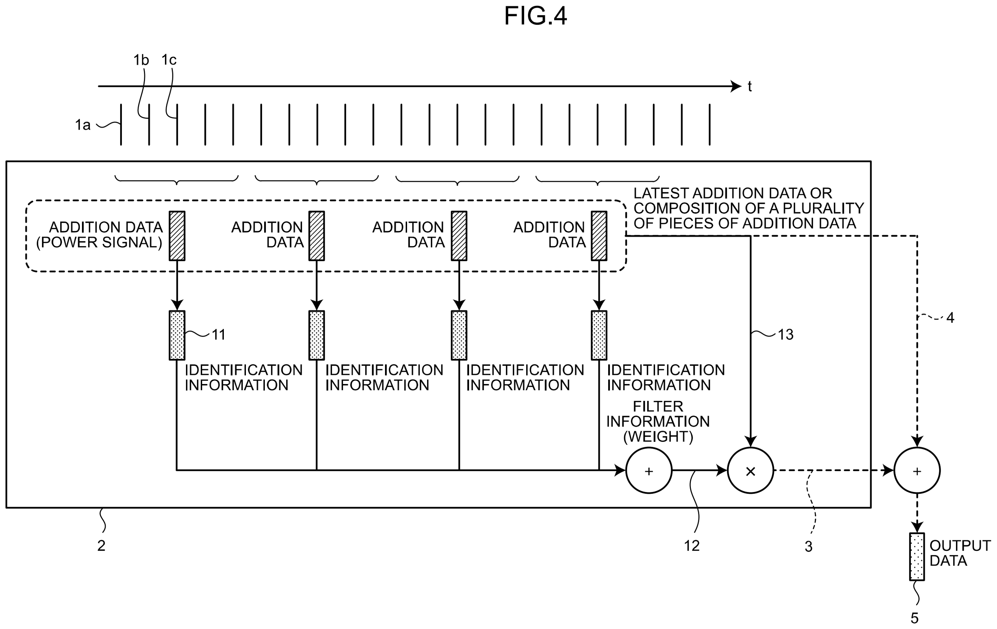

Next, the execution function 150b executes high-definition processing 2 that locally extracts or emphasizes the fluid represented by the first ultrasound data to acquire the second ultrasound data (Step S130). As illustrated in FIG. 4, the execution function 150b generates addition data (data representing a single image) by adding/time-averaging (persistence) the pieces of frame data 1a, 1b, 1c, and the like that are consecutive in the time direction and for which a clutter component has been reduced by the processing at Step S110 every certain number of frames (FIG. 4 illustrates a case of five frames).The certain number of frames (number of packets) referred to here is actually, for example, about a few frames to a hundred frames.

The addition data has a higher signal-to-noise ratio (SN ratio) than each frame data 1a, 1b, or 1c. The addition data is, for example, blood flow data (power Doppler data) in which the blood flow-derived information is emphasized. The addition data corresponds to a single image in which the signal of the fluid after clutter reduction is averaged over a certain time to represent the boundaries of flow paths (for example, the shape of blood vessels). The addition data includes signal values (amplitude values) representing speckle patterns caused by strengthening interference and weakening interference between ultrasound waves reflected by the fluid, and signal values representing other noise (artifacts and the like) that occur instantaneously or intermittently on the time axis.

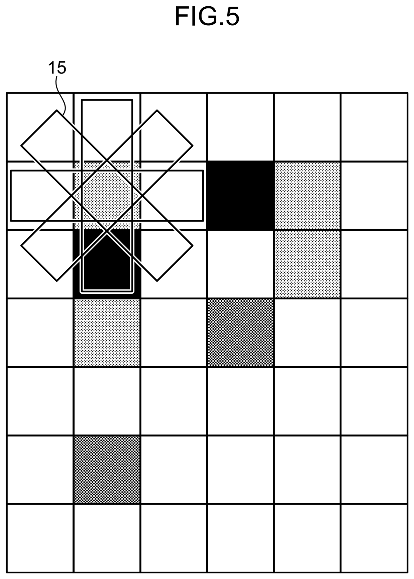

The execution function 150b sequentially generates filter information 12 representing a local peak position of the speckle pattern using the addition data of a plurality of frames. Specifically, for each position of the addition data for the four frames illustrated in FIG. 4, information (identification information 11) identifying the magnitude relation between the signal value corresponding to that position and the signal value corresponding to the surroundings of that position is acquired. For example, as illustrated in FIG. 5, the execution function 150b places a kernel 15 at a position of interest in the frame (image) represented by the addition data, and compares the magnitude relation between the signal value corresponding to the position of interest and the signal value corresponding to each position in the range in which the kernel 15 is placed (around the position of interest).The execution function 150b, for example, acquires, for each position in the frame, information that identifies whether the signal value is larger than the signal value corresponding to the surroundings of that position as the identification information. In other words, the identification information 11 can also be said to be information that identifies a position where the signal value is relatively large (the local peak position) in a local range of the frame.

Specifically, when using the kernel 15 based on a ratio of 3 vertical by 1 horizontal, the execution function 150b places the center of the kernel 15 (the second square) at the position of interest, and identifies whether the signal value corresponding to that position is larger than the signal values corresponding to the positions at both ends of the kernel 15 (the first square and the third square). For example, the execution function 150b represents "1" when the signal value corresponding to the position of interest is larger than the signal values corresponding to the surroundings of that position, and represents "0" when the signal value corresponding to that position is smaller than the signal values corresponding to the surroundings of that position. The execution function 150b extracts the identification information for each position of the image (frame) represented by the addition data, thereby extracting the identification information 11 for the entire image (frame) represented by the addition data. That is, this extraction processing converts the addition data into a binary image in which each pixel is represented by "0" or "1."

Note that the example in FIG. 5 illustrates an example of extracting the identification information 11 by arranging the kernel 15 in four directions (vertical, horizontal, diagonally upper right, and diagonally upper left) for each position of the frame represented by the addition data, but the shape, size, and arrangement of the kernel are not limited to this example. The example in FIG. 5 illustrates an example of extracting an object (fluid) in pixels, but the identification information may be extracted for each position of the frame in groups having a plurality of pixels. When the identification information is extracted in groups having the pixels, the average or integrated value of the signal values of the positions constituting the group may be used as a signal value corresponding to each position of that frame.

Furthermore, the execution function 150b generates the filter information 12 based on a plurality of pieces of identification information 11. The filter information 12 is information statistically representing a local fluid peak position in individual frames, within a time corresponding to the frames. The local fluid peak position is a position at which the signal value is relatively high in a local range of a frame. For example, the execution function 150b generates the filter information 12 using the identification information 11 for four frames. The filter information 12 is determined in accordance with a value represented by each identification information 11 at a position (coordinates (x, y)) corresponding to each other between the frames. For example, if the four frames are all "1" at the position corresponding to each other of each identification information 11, the filter information at that position is "1." If "1" is obtained three times and "0" is obtained once out of the four frames at the position corresponding to each other of each identification information 11, the filter information at that position is "0.75." If "1" is obtained twice and "0" is obtained twice out of the four frames at the position corresponding to each other of each identification information 11, the filter information at that position is "0.5." If "1" is obtained once and "0" is obtained three times out of the four frames at the position corresponding to each other of each identification information 11, the filter information at that position is "0.25." If no "1" is obtained and "0" is obtained four times out of the four frames at the position corresponding to each other of each identification information 11, the filter information at that position is "0."

Thus, in the identification information 11 for a plurality of frames, as the number of times "1" is obtained at the position corresponding to each becomes larger, a larger signal value is continuously obtained at that position compared to the surrounding area, and it is more likely that the fluid is present, and thus the filter information 12 corresponding to that position is set to a larger value (for example, "1," "0.75," and "0.5"). On the other hand, in the identification information 11 for the frames, as the number of times "0" is obtained at the position corresponding to each other becomes larger, it is less likely that the fluid is present at that position, and thus the filter information 12 corresponding to that position is set to a smaller value (for example, "0.25" and "0").

The execution function 150b generates the second ultrasound data based on the filter information 12 and at least one piece of addition data 13 of a plurality of pieces of addition data. Specifically, the execution function 150b generates the second ultrasound data by using the latest addition data among the pieces of addition data or composite addition data obtained by composing the pieces of addition data with each other as the addition data 13, and multiplying a signal value (an amplitude value) representing each position of the frame represented by the addition data 13 by the filter information (a weight factor) set for each corresponding position of the filter information 12. In other words, the second ultrasound data is data obtained by converting the addition data 13 by the filter information 12. In the second ultrasound data, a position in the addition data 13 where it is likely that the fluid is present is multiplied by the weight factor of a larger value by the filter information 12, and the position of the fluid is emphasized. On the other hand, in the second ultrasound data, a position in the addition data 13 where it is likely that noise (artifacts or the like) generated instantaneously or intermittently is present is multiplied by the weight factor of a smaller value by the filter information 12, and the noise is reduced. In this way, the execution function 150b executes the high-definition processing to acquire the second ultrasound data.

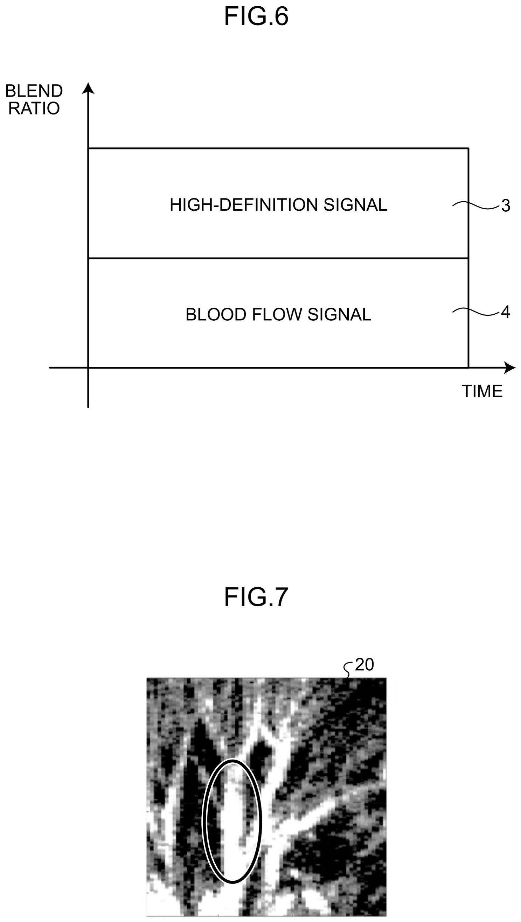

Next, the composition function 150c composites a first ultrasound image 4 based on the first ultrasound data and a second ultrasound image 3 based on the second ultrasound data with each other to generate a composite image (Step S140). For example, the first ultrasound image 4 based on the first ultrasound data is an image based on the addition data 13 (a blood flow signal) obtained by performing the processing described above on the first ultrasound data, and the second ultrasound image 3 based on the second ultrasound data is an image based on the second ultrasound data (a high-definition signal). As illustrated in FIG. 6, the composition function 150c composites the first ultrasound image 4 based on the blood flow signal and the second ultrasound image 3 based on the high-definition signal with each other at a certain composition ratio to generate a composite image 5.

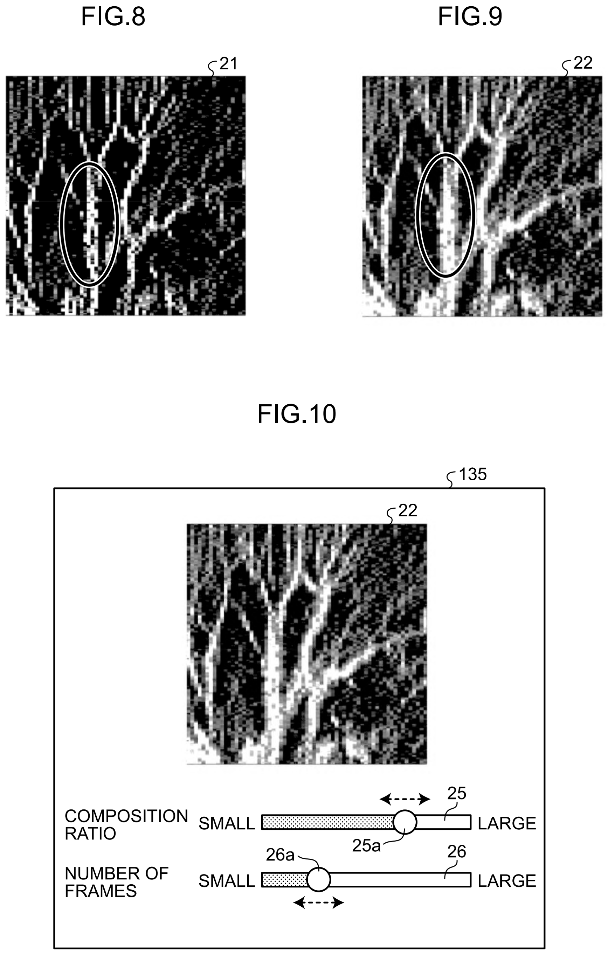

The display control function 150d displays the composite image 5 obtained by the composition function 150c on the display 135 (Step 150). FIG. 7 illustrates a blood flow image 20 (the first ultrasound image 4 illustrated in FIG. 3 and FIG. 4), FIG. 8 illustrates a high-definition image 21 (the second ultrasound image 3 illustrated in FIG. 3 and FIG. 4), and FIG. 9 illustrates a composite image 22 (the composite image 5 illustrated in FIG. 3 and FIG. 4). The high-definition image 21 (the second ultrasound image 3) has improved visibility (contrast and sharpness) of blood flow compared to the blood flow image 20 (the first ultrasound image 4). However, when the real-time properties of the high-definition processing 2 are emphasized, the position of the local peak signal may become discontinuous, and the connection of blood flow may not be able to be expressed. For example, in the thick blood vessel (the oval range) illustrated in the blood flow image 20 in FIG. 7, the connection of blood flow is not expressed at the corresponding position in the high-definition image 21 in FIG. 8. On the other hand, the connection of blood flow is expressed at the corresponding position in the composite image 22 in FIG. 9.

As described above, the present embodiment sequentially acquires the first ultrasound data representing the fluid in the body of the subject in the time direction, executes the high-definition processing that locally extracts or emphasizes the fluid represented by the first ultrasound data to acquire the second ultrasound data, and composites the first ultrasound image based on the first ultrasound data and the second ultrasound image based on the second ultrasound data with each other. This can reduce the problem of trade-off caused by the processing that improves the image quality of the ultrasound image.

Note that the above embodiment describes a case in which the high-definition processing is performed using the 1 × 3 kernel 15, but the embodiment is not limited to this example. For example, using a 3 × 3-pixel size kernel, a local peak may be extracted by processing that extracts a pixel value if the pixel at the center position of interest is larger than the surrounding pixels (eight pixels).

In the high-definition processing at Step S130, instead of the kernel 15, the identification information 11 for the frames may be acquired by extracting signal values that are larger than a preset threshold.

The above embodiment describes an example in which the pieces of frame data 1a, 1b, 1c, ... consecutive in the time direction are added/time-averaged (persistence) every certain number of frames to generate the addition data, and the identification information 11 is acquired from the addition data, but the identification information 11 may be acquired from each of the pieces of frame data 1a, 1b, 1c, ... without being acquired from the addition data.

The execution function 150b may execute the high-definition processing 2 using, for example, an artificial intelligence (AI) model obtained by neural networks or machine learning. In this case, the execution function 150b may input the pieces of frame data 1a, 1b, 1c, ... (the first ultrasound data) that are consecutive in the time direction and for which the clutter component has been reduced by the processing at Step S110 into the AI model every certain number of frames (for example, 30 frames), and acquire high-definition data output by the AI model as the second ultrasound data. The execution function 150b may input the addition data for a plurality of frames (FIG. 4 illustrates a case of four frames) that have been added/time-averaged every certain number of frames (FIG. 4 illustrates a case of five frames) into the AI model, and acquire high-definition data output by the AI model as the second ultrasound data.

Similarly, the composition function 150c may input the pieces of frame data 1a, 1b, 1c, ... (the first ultrasound data) consecutive in the time direction into the AI model every certain number of frames (for example, 30 frames), and acquire an image based on the first ultrasound data output by the AI model as the first ultrasound image 4. The composition function 150c may input the second ultrasound data into the AI model and acquire an image based on the second ultrasound data output by the AI model as the second ultrasound image 3.

The present embodiment describes an example of a case in which the composition function 150c composites the first ultrasound image 4 and the second ultrasound image 3 with each other at a certain composition ratio, but this is not limiting. For example, the composition ratio may be adjustable by a user's operation via the input apparatus 134. Specifically, as illustrated in FIG. 10, the display control function 150d may display a graphical user interface (GUI) including two types of sliders 25 and 26 on the display 135 in addition to the composite image 22.

In this case, the slider 25 receives an operation to adjust the composition ratio at which the blood flow image 20 (the first ultrasound image 4 illustrated in FIG. 3 and FIG. 4) and the high-definition image 21 (the second ultrasound image 3 illustrated in FIG. 3 and FIG. 4) are composited with each other. The user performs an operation to move a knob 25a in the slider 25 to the left or right via the input apparatus 134. In the slider 25 illustrated in FIG. 10, as the knob 25a is moved to the right more, the composition ratio of the blood flow image 20 (the first ultrasound image 4) to the high-definition image 21 (the second ultrasound image 3) becomes larger. The composition function 150c composites the blood flow image 20 and the high-definition image 21 with each other based on the composition ratio received by the slider 25. This causes the display 135 to display the composite image 22 generated at the composition ratio adjusted in accordance with the user's operation.

The slider 26 receives an operation to adjust the number of analysis frames (an analysis time) of the high-definition processing 2. As described above, in the high-definition processing 2, as the number of analysis frames becomes larger (as the analysis time becomes longer), the position of the local peak signal can be represented to be more continuous. The user performs an operation to move a knob 26a in the slider 26 to the left or right via the input apparatus 134. In the slider 26 illustrated in FIG. 10, as the knob 26a is moved to the right more, the number of analysis frames becomes larger. The number of analysis frames to be adjusted is at least either the number of frames for which addition/time-averaging (persistence) is performed on the frame data 1a, 1b, and 1c (the first ultrasound data) after the clutter component has been reduced (FIG. 4 illustrates a case of five frames) or the number of frames of the addition data used to generate the filter information 12 (FIG. 4 illustrates a case of four frames). The execution function 150b executes the high-definition processing 2 based on the number of analysis frames received by the slider 26 to acquire the second ultrasound data. This enables the image quality of the second ultrasound image 3 based on the second ultrasound data to be adjusted. Thus, the image quality of the ultrasound image can be adjusted by a combination of the two types of sliders 25 and 26.

### Second Embodiment

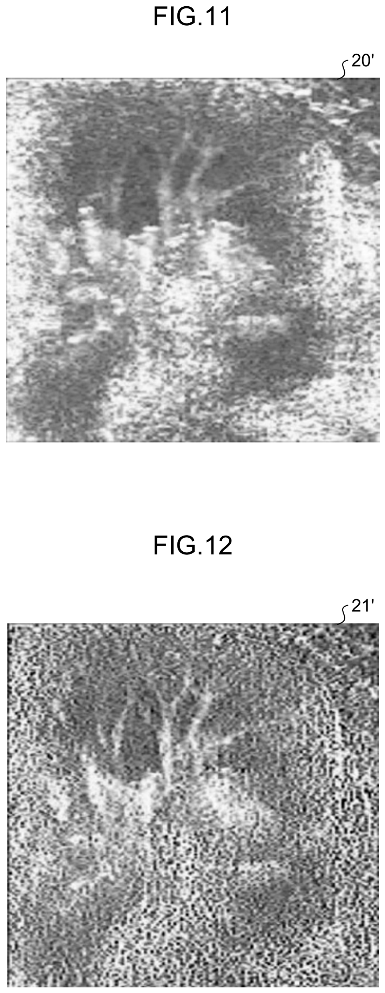

The first embodiment describes an example of a case in which the composition ratio at which the first ultrasound image 4 and the second ultrasound image 3 are composited with each other is fixed, but this is not limiting. For example, even when the filter processing is performed by the acquisition function 150a, a clutter component may remain in the first ultrasound data acquired by the filter processing. When the high-definition processing 2 is executed on the first ultrasound data in which the clutter component remains, the image quality of the ultrasound image based on the second ultrasound data (the second ultrasound image) may be degraded (the connection of blood flow cannot be expressed) because the extraction or emphasis of the local peak signal is not performed properly. For example, a blood flow image 20' (the first ultrasound image) obtained when the clutter component remains in the first ultrasound data is affected by the clutter, as illustrated in FIG. 11. When the high-definition processing 2 is executed on such first ultrasound data, the connection of blood flow cannot be expressed in a high-definition image 21' (the second ultrasound image), as illustrated in FIG. 12. The present embodiment describes an example of a case in which a composite image is generated adaptively in accordance with the way clutter appears.

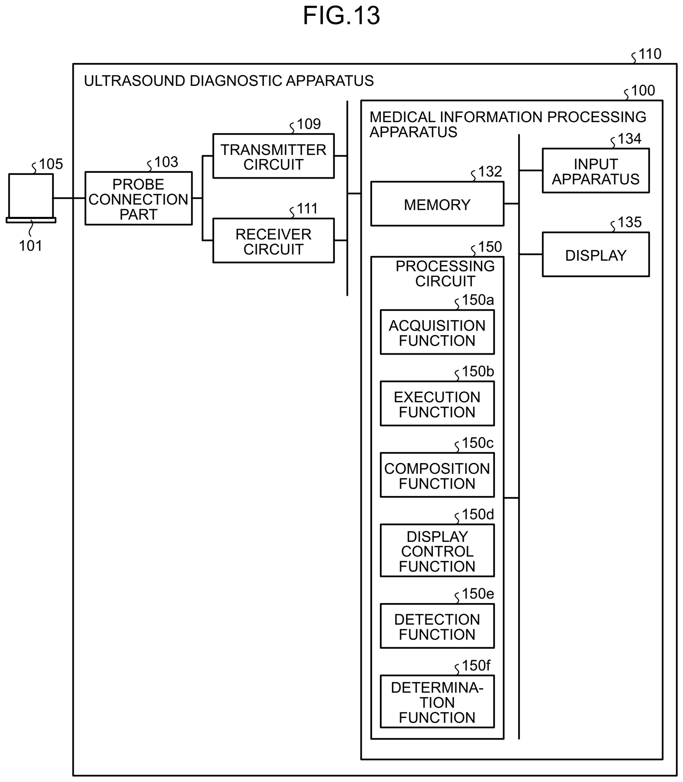

An example of the configuration of a medical information processing apparatus according to a second embodiment will be described using FIG. 13. For substantially the same configurations as those of the medical information processing apparatus according to the first embodiment, duplicated descriptions will be omitted as appropriate. The processing circuit 150 executes computer programs stored in the memory 132, thereby functioning as the acquisition function 150a, the execution function 150b, the composition function 150c, the display control function 150d, a detection function 150e, and a determination function 150f. The acquisition function 150a, the execution function 150b, the composition function 150c, the display control function 150d, the detection function 150e, and the determination function 150f are examples of the acquisition unit, the execution unit, the composition unit, the display controller, a detector, and a determination unit, respectively.

The detection function 150e detects a characteristic amount of clutter. The determination function 150f determines the composition ratio at which the first ultrasound data and the second ultrasound data are composited with each other based on the characteristic amount of clutter. The composition function 150c composites the first ultrasound image (the blood flow image 20) and the second ultrasound image (the high-definition image 21) with each other based on the composition ratio determined by the determination function 150f to generate the composite image 22.

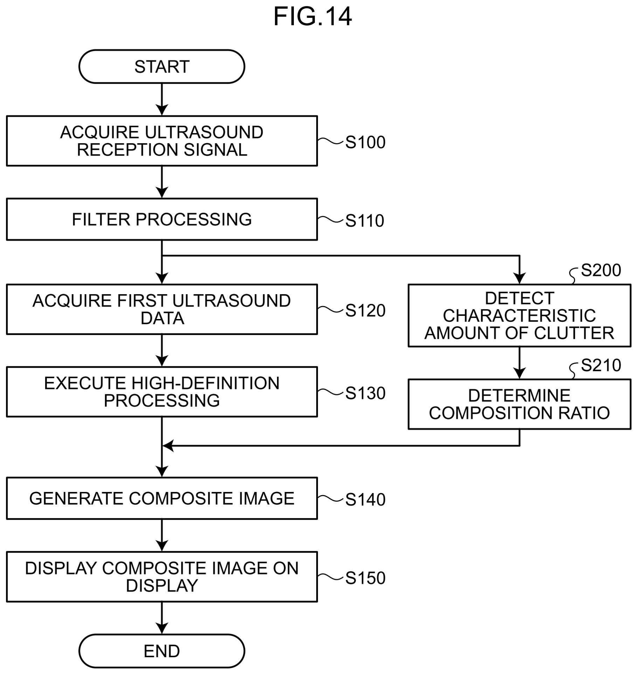

The details of the operation of the ultrasound diagnostic apparatus 110 will be described below with reference to FIG. 14 to FIG. 19. FIG. 14 is a flowchart illustrating the flow of processing performed by the medical information processing apparatus 100 according to the second embodiment.

The detection function 150e detects the characteristic amount of clutter from the information obtained during the filter processing at Step S110. For example, for the filter processing, an adaptive filter that reduces clutter contained in the input signal is used. The adaptive filter changes a filter coefficient in accordance with the input signal using eigenvalue decomposition, singular value decomposition, or the like. The detection function 150e detects the filter coefficient of the adaptive filter as the characteristic amount of clutter (Step S200). For example, in the case of eigenvalue decomposition, the adaptive filter decomposes the input signal into a plurality of eigenvalues. Each eigenvalue is a filter coefficient, which changes in accordance with the input signal.

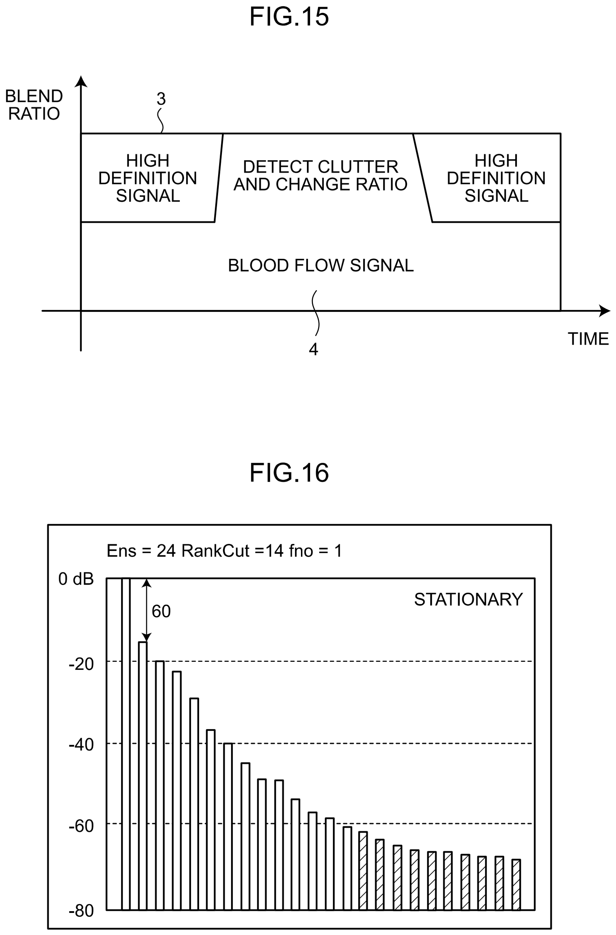

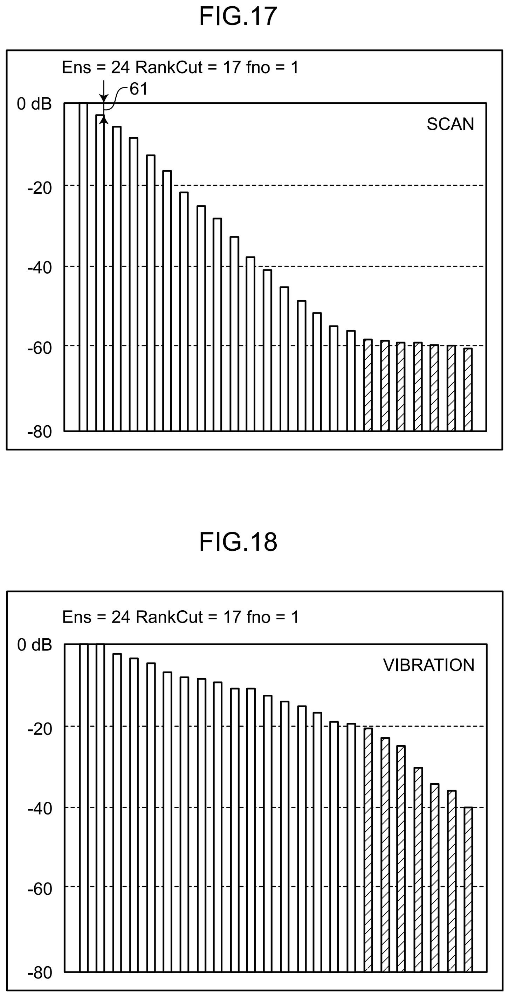

For example, FIG. 16 to FIG. 18 illustrate the distribution of the filter coefficient (the eigenvalues) for different clutter amounts contained in the input signal (frame data to be filtered). As illustrated in FIG. 16 to FIG. 18, the eigenvalues, the difference between the eigenvalues, and the distribution of the filter coefficient (the eigenvalues) change in accordance with the clutter amount contained in the input signal. The clutter amount contained in the input signal when the distribution of the filter coefficient illustrated in FIG. 16 is obtained is smaller than the clutter amount contained in the input signal when the distribution of the filter coefficient illustrated in FIG. 17 is obtained. The clutter amount contained in the input signal when the distribution of the filter coefficient illustrated in FIG. 17 is obtained is smaller than the clutter amount contained in the input signal when the distribution of the filter coefficient illustrated in FIG. 18 is obtained.

In the distribution of the filter coefficient, each of the eigenvalues is given a number (an eigenvalue number), and a graph of length corresponding to the eigenvalue is illustrated in the order of the eigenvalue number. Specifically, they are referred to as a first eigenvalue, a second eigenvalue, ... in ascending order of the eigenvalue number. The graph illustrated in the leftmost position in FIG. 16 to FIG. 18 illustrates the first eigenvalue, and the graph illustrated in the position next to that graph illustrates the second eigenvalue. A difference 60 between the first eigenvalue and the second eigenvalue illustrated in FIG. 16 is smaller than a difference 61 between the first eigenvalue and the second eigenvalue illustrated in FIG. 17. As illustrated in FIG. 16 to FIG. 18, it can be seen that a smaller clutter amount contained in the input signal gives a larger difference between the eigenvalues. As illustrated in FIG. 16 to FIG. 18, the magnitude of the clutter amount can also be identified by the shape illustrating the distribution of the filter coefficient (the shape of the slope).

The detection function 150e detects the distribution of the eigenvalues decomposed by the adaptive filter as the characteristic amount of clutter. Specifically, the detection function 150e detects the difference between two eigenvalues (for example, the first eigenvalue and the second eigenvalue) out of the eigenvalues as the characteristic amount of clutter.

The determination function 150f then determines the composition ratio based on the filter coefficient detected by the detection function 150e (Step S210). Specifically, the determination function 150f determines the composition ratio in accordance with the difference between the two eigenvalues (for example, the first eigenvalue and the second eigenvalue) detected by the detection function 150e. For example, the composition ratio is determined such that the ratio of the first ultrasound image (the blood flow image 20) to the second ultrasound image (the high-definition image 21) becomes larger as the difference between the two eigenvalues becomes smaller.

As illustrated in FIG. 15, the composition function 150c composites the first ultrasound image (the blood flow image 20) and the second ultrasound image (the high-definition image 21) with each other based on the composition ratio adaptively adjusted and determined by the determination function 150f to generate the composite image 22 (Step S140).

As described above, the present embodiment can adaptively generate a composite image in accordance with the way clutter appears by determining the composition ratio at which the first ultrasound data and the second ultrasound data are composited with each other based on the characteristic amount of clutter.

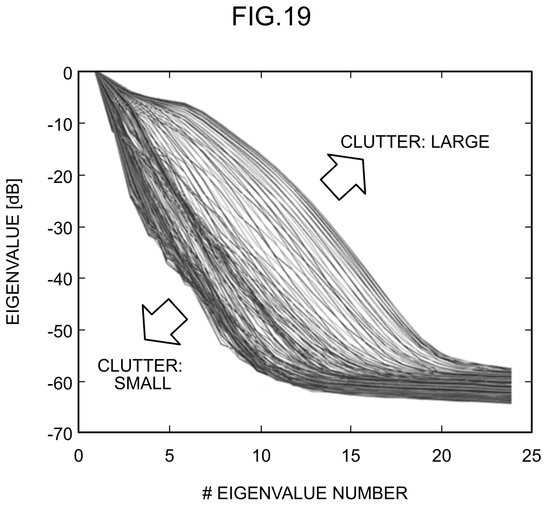

Note that the present embodiment describes an example of a case in which the detection function 150e detects the difference between the first eigenvalue and the second eigenvalue among the eigenvalues decomposed by the adaptive filter as the characteristic amount of clutter, but this is not limiting. For example, the detection function 150e may detect the difference between the first eigenvalue and a 10th eigenvalue among the eigenvalues as the characteristic amount of clutter. The detection function 150e may detect the statistics of the difference between the eigenvalues as the characteristic amount of clutter. In addition, the detection function 150e may detect the slope of the eigenvalues illustrated in FIG. 16 to FIG. 18 as the characteristic amount of clutter. As illustrated in FIG. 19, the eigenvalue may be plotted against the eigenvalue number. When clutter is large, the eigenvalue tends not to decay even though the eigenvalue number becomes large. The detection function 150e may detect the position and shape of the eigenvalue plotted against the eigenvalue number as the characteristic amount of clutter.

The present embodiment describes an example of a case in which the characteristic amount of clutter is determined from the eigenvalues decomposed by the adaptive filter, but this is not limiting. For example, the detection function 150e may detect the signal intensity of at least one ultrasound data of the first ultrasound data and the second ultrasound data as the characteristic amount of clutter. For example, when many clutter components remain in the first ultrasound data (power Doppler data) obtained by the filter processing at Step S110, the signal intensity of the first ultrasound data is larger than the signal intensity of the first ultrasound data when fewer clutter components remain. For example, the detection function 150e may detect the average of signal intensity within the frame represented by the first ultrasound data as the characteristic amount of clutter. The detection function 150e may detect the average of signal intensity within the frame represented by the second ultrasound data as the characteristic amount of clutter. In this case, the determination function 150f determines the composition ratio based on the signal intensity (the characteristic amount of clutter) of at least one ultrasound data of the first ultrasound data and the second ultrasound data detected by the detection function 150e.

The detection function 150e may detect a temporal change in the signal intensity of at least one ultrasound data of the first ultrasound data and the second ultrasound data as the characteristic amount of clutter. For example, when the signal intensity of the first ultrasound data (power Doppler data) obtained by the filter processing at Step S110 significantly changes in the time direction (a frame direction), the first ultrasound data has more residual clutter components than the first ultrasound data obtained before and after it. By detecting the temporal change in the signal intensity of at least one ultrasound data of the first ultrasound data and the second ultrasound data as the characteristic amount of clutter, a frame in which clutter is present is efficiently detected. In this case, the determination function 150f determines the composition ratio based on the temporal change in the signal intensity of at least one ultrasound data of the first ultrasound data and the second ultrasound data detected by the detection function 150e (the characteristic amount of clutter).

The detection function 150e may detect a temporal change in the phase information (a velocity difference) of at least one ultrasound data of the first ultrasound data and the second ultrasound data as the characteristic amount of clutter. When the phase information (velocity) of the first ultrasound data (power Doppler data) obtained by the filter processing at Step S110 significantly changes in the time direction (the frame direction), the first ultrasound data has more residual clutter components than the first ultrasound data obtained before and after it. By detecting the temporal change in the phase information of at least one ultrasound data of the first ultrasound data and the second ultrasound data as the characteristic amount of clutter, a frame in which clutter is present is efficiently detected. In this case, the determination function 150f determines the composition ratio based on the temporal change in the phase information of at least one ultrasound data of the first ultrasound data and the second ultrasound data detected by the detection function 150e (the characteristic amount of clutter).

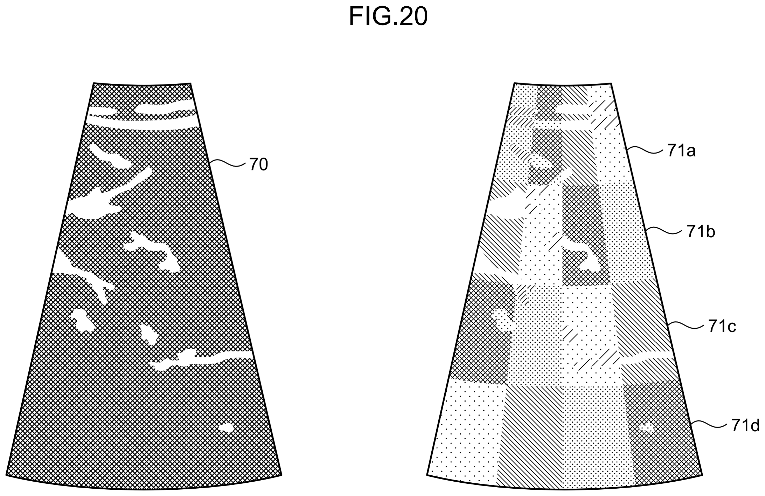

Furthermore, the detection function 150e may detect the characteristic amount of clutter for each of the positions of the frames in at least one ultrasound data of the first ultrasound data and the second ultrasound data. Specifically, as illustrated in FIG. 20, the detection function 150e spatially divides the frame of at least one ultrasound data of the first ultrasound data and the second ultrasound data into a plurality of regions, and detects the characteristic amount of clutter for each of the spatially divided regions. For example, a region 70 illustrated in the left figure in FIG. 20 is a normal ROI, and regions 71a, 71b, 71c, and 71d illustrated in the right figure in FIG. 20 are the spatially divided regions. The detection function 150e detects the characteristic amount of clutter for the spatially divided regions 71a, 71b, 71c, and 71d. Furthermore, the detection function 150e may detect the characteristic amount of clutter of each region by analyzing the spatially divided regions 71a, 71b, 71c, and 71d in the time direction. In this case, the determination function 150f, based on the characteristic amount of clutter detected for each of the positions by the detection function 150e, determines the composition ratio of each position. The composition function 150c performs composition of the first ultrasound image (the blood flow image 20) and the second ultrasound image (the high-definition image 21) at each position based on the composition ratio of each position determined by the determination function 150f.

Furthermore, the above embodiment describes an example of a case in which the processing at Step S140, which generates the composite image, is always executed, but this is not limiting. For example, the composition function 150c may control whether the first ultrasound image (the blood flow image 20) and the second ultrasound image (the high-definition image 21) are composited with each other based on the characteristic amount of clutter detected by the detection function 150e. For example, when the clutter amount is large (when it exceeds a threshold), the composition function 150c composites the first ultrasound image (the blood flow image 20) and the second ultrasound image (the high-definition image 21) with each other. On the other hand, when the clutter amount is small (when it is the threshold or less), the composition function 150c does not composite the first ultrasound image (the blood flow image 20) and the second ultrasound image (the high-definition image 21) with each other. The display control function 150d displays the composite image when the first ultrasound image (the blood flow image 20) and the second ultrasound image (the high-definition image 21) are composited with each other by the composition function 150c, and displays the second ultrasound image (the high-definition image 21) when the first ultrasound image (the blood flow image 20) and the second ultrasound image (the high-definition image 21) are not composited with each other by the composition function 150c.

Note that when the clutter amount is small (when it is the threshold or less), the composition function 150c may composite the first ultrasound image (the blood flow image 20) and the second ultrasound image (the high-definition image 21) with each other, while when the clutter amount is large (when it exceeds the threshold), the composition function 150c may not composite the first ultrasound image (the blood flow image 20) and the second ultrasound image (the high-definition image 21) with each other. In this case, the display control function 150d may display the composite image when the first ultrasound image (the blood flow image 20) and the second ultrasound image (the high-definition image 21) are composited with each other by the composition function 150c, and may display the first ultrasound image (the blood flow image 20) when the first ultrasound image (the blood flow image 20) and the second ultrasound image (the high-definition image 21) are not composited with each other by the composition function 150c.

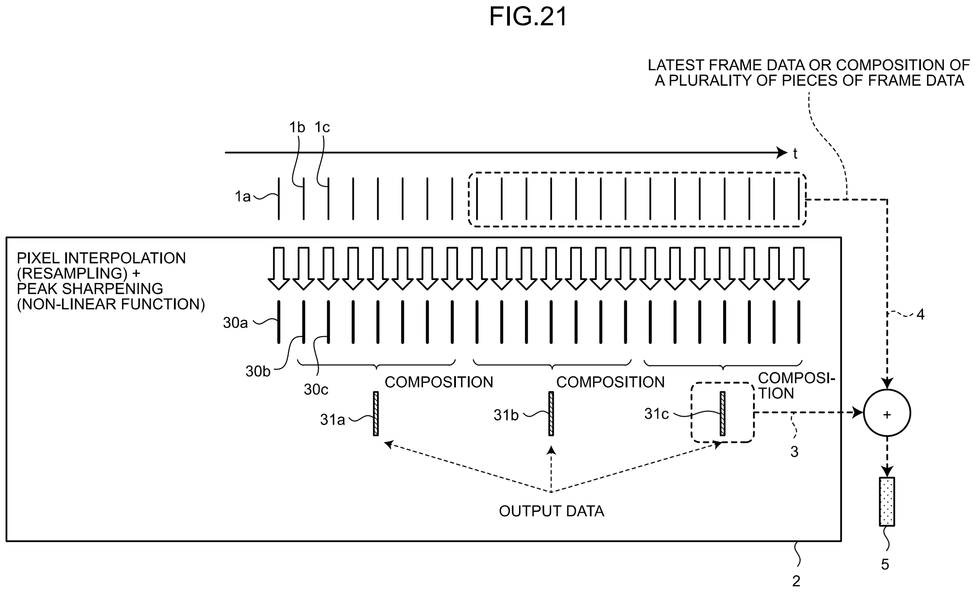

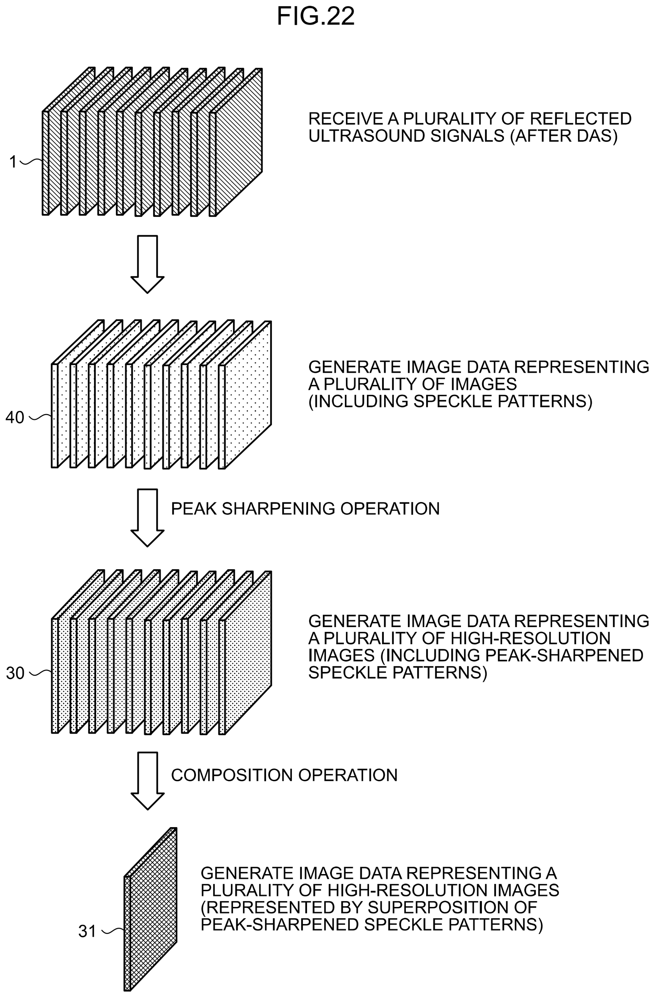

In addition, the above embodiment describes an example of the high-definition processing 2 illustrated in FIG. 4, but as another example, the high-definition processing 2 illustrated in FIG. 21 and FIG. 22 may be performed. For example, as illustrated in FIG. 21, the acquisition function 150a acquires the first ultrasound data representing the fluid in the body of the subject based on the ultrasound reception signal received by the ultrasound probe 105. Specifically, when the filter processing at Step S110 is performed, the acquisition function 150a sequentially acquires a plurality of pieces of frame data 1a, 1b, 1c, ... consecutive in the time direction as the first ultrasound data (Step S120). Each frame data is power Doppler data and contains an amplitude value (a power signal value) of a blood flow signal (a fluid signal) corresponding to each pixel constituting one image (frame).

Next, the execution function 150b performs processing that enhances image density on the frame data 1a, 1b, 1c, ... to generate image data 40 representing a plurality of images. The execution function 150b resamples the image data to generate additional interpolation pixels in the image data, thereby performing processing that enhances image density to generate the image data 40 representing the images. The image data 40 representing the images referred to in this example includes speckle patterns.

The execution function 150b then performs a peak sharpening operation on the image data 40 to generate image data 30 representing a plurality of resolution images. As the peak sharpening operation, for example, known interpolation methods and nonlinear transformation using power functions or the like are used. As an example, the execution function 150b performs pixel interpolation (resampling) processing and peak sharpening processing using a nonlinear function on each of the frame data 1a, 1b, 1c, ... to generate data 30a, 30b, 30c, and the like, as illustrated in FIG. 21. Here, the image data 30 contains peak-sharpened speckle patterns.

The execution function 150b then performs a composition operation on the image data 30 to generate data 31 representing a plurality of high-resolution images. For example, the execution function 150b composites the data 30a, 30b, 30c, and the like to generate data 31a. The execution function 150b composites data 31b, 31c, and the like in a similar manner. The data 31 is represented by a superposition of the peak-sharpened speckle patterns. The execution function 150b generates the thus obtained data 31 as the second ultrasound data in which the high-definition processing 2 has been executed. Thus, the high-definition processing 2 includes the processing that enhances pixel density, the peak sharpening operation, and the composition operation. Also in this case, when the real-time properties of the high-definition processing are emphasized, the position of the local peak signal (a peak sharpening signal) becomes discontinuous, and thus the image quality of the second ultrasound image 3 (the high-definition image) based on the second ultrasound data (the data 31) will be degraded (the connection of blood flow cannot be expressed).

Given this, the composition function 150c composites the first ultrasound image 4 (the blood flow image) based on the first ultrasound data and the second ultrasound image 3 (the high-resolution image) based on the second ultrasound data with each other to generate the composite image 5. This reduces the problem of trade-off described above caused by the processing that improves the image quality of the ultrasound image.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. An ultrasound diagnostic apparatus (110) comprising a processing circuit (150) configured to
sequentially acquire first ultrasound data (1a, 1b, 1c) representing a fluid in a body of a subject in a time direction based on an ultrasound reception signal received by an ultrasound probe (105),
execute high-definition processing that locally extracts or emphasizes the fluid represented by the first ultrasound data (1a, 1b, 1c) to acquire second ultrasound data,
composite a first ultrasound image (4) that is based on the first ultrasound data (1a, 1b, 1c) and a second ultrasound image (3) that is based on the second ultrasound data, and
display a composite image (5) obtained by the composition.

2. The ultrasound diagnostic apparatus (110) according to claim 1, wherein the processing circuit (150) is configured to
detect a characteristic amount of clutter,
determine a composition ratio at which the first ultrasound data (1a, 1b, 1c) and the second ultrasound data are composited with each other based on the characteristic amount of clutter, and
composite the first ultrasound image (4) and the second ultrasound image (3) with each other based on the determined composition ratio.

3. The ultrasound diagnostic apparatus (110) according to claim 2, wherein the processing circuit (150) is configured to
detect a filter coefficient of an adaptive filter as the characteristic amount of clutter, the adaptive filter reducing clutter contained in an input signal, and
determine the composition ratio based on the detected filter coefficient.

4. The ultrasound diagnostic apparatus (110) according to claim 3, wherein
the adaptive filter is configured to decompose the input signal into a plurality of eigenvalues, and
the processing circuit (150) is configured to detect distribution of the eigenvalues decomposed by the adaptive filter as the characteristic amount of clutter.

5. The ultrasound diagnostic apparatus (110) according to claim 4, wherein the processing circuit (150) is configured to
detect a difference between two eigenvalues among the eigenvalues as the characteristic amount of clutter, and
determine the composition ratio such that a ratio of the first ultrasound image (4) to the second ultrasound image (3) becomes larger as the difference between the eigenvalues becomes smaller.

6. The ultrasound diagnostic apparatus (110) according to claim 2, wherein the processing circuit (150) is configured to detect signal intensity of at least one ultrasound data of the first ultrasound data (1a, 1b, 1c) and the second ultrasound data as the characteristic amount of clutter.

7. The ultrasound diagnostic apparatus (110) according to claim 2, wherein the processing circuit (150) is configured to detect a temporal change in signal intensity of at least one ultrasound data of the first ultrasound data (1a, 1b, 1c) and the second ultrasound data as the characteristic amount of clutter.

8. The ultrasound diagnostic apparatus (110) according to claim 2, wherein the processing circuit (150) is configured to detect a temporal change in phase information of at least one ultrasound data of the first ultrasound data (1a, 1b, 1c) and the second ultrasound data as the characteristic amount of clutter.

9. The ultrasound diagnostic apparatus (110) according to claim 2, wherein the processing circuit (150) is configured to
detect the characteristic amount of clutter for each of positions of frames in at least one ultrasound data of the first ultrasound data (1a, 1b, 1c) and the second ultrasound data,
determine the composition ratio of each of the positions based on the characteristic amount of clutter detected for each position; and
perform composition of the first ultrasound image (4) and the second ultrasound image (3) at each position based on the determined composition ratio of each position.

10. The ultrasound diagnostic apparatus (110) according to claim 1, wherein
the processing circuit (150) is configured to receive an operation to adjust a composition ratio at which the first ultrasound image (4) and the second ultrasound image (3) are composited with each other, and
the processing circuit (150) is configured to composite the first ultrasound image (4) and the second ultrasound image (3) with each other based on the received composition ratio.

11. The ultrasound diagnostic apparatus (110) according to claim 1, wherein the processing circuit (150) is configured to
detect a characteristic amount of clutter,
control whether the first ultrasound image (4) and the second ultrasound image (3) are composited with each other based on the characteristic amount of clutter, and
display the composite image (5) when the first ultrasound image (4) and the second ultrasound image (3) are composited with each other and display the second ultrasound image (3) when the first ultrasound image (4) and the second ultrasound image (3) are not composited with each other by the composition unit.

12. The ultrasound diagnostic apparatus (110) according to claim 1, wherein the processing circuit (150) is configured to
detect a characteristic amount of clutter,
control whether the first ultrasound image (4) and the second ultrasound image (3) are composited with each other based on the characteristic amount of clutter, and
display the composite image (5) when the first ultrasound image (4) and the second ultrasound image (3) are composited with each other and display the first ultrasound image (4) when the first ultrasound image (4) and the second ultrasound image (3) are not composited with each other.

13. A medical information processing apparatus comprising a processing circuit (150) configured to
sequentially acquire first ultrasound data (1a, 1b, 1c) representing a fluid in a body of a subject in a time direction based on an ultrasound reception signal received by an ultrasound probe (105),
execute high-definition processing that locally extracts or emphasizes the fluid represented by the first ultrasound data (1a, 1b, 1c) to acquire second ultrasound data,
composite a first ultrasound image (4) that is based on the first ultrasound data (1a, 1b, 1c) and a second ultrasound image (3) that is based on the second ultrasound data, and
display an obtained composite image (5).

14. A medical information processing method comprising:
sequentially acquiring, by a computer, first ultrasound data (1a, 1b, 1c) representing a fluid in a body of a subject in a time direction based on an ultrasound reception signal received by an ultrasound probe (105),
executing, by the computer, high-definition processing that locally extracts or emphasizes the fluid represented by the first ultrasound data (1a, 1b, 1c) to acquire second ultrasound data;
composing, by the computer, a first ultrasound image (4) that is based on the first ultrasound data (1a, 1b, 1c) and a second ultrasound image (3) that is based on the second ultrasound data; and
displaying, by the computer, a composite image (5).
